# EUROPEAN PATENT SPECIFICATION

(11) **EP 0 888 783 B1**
(45) Date of publication and mention of the grant of the patent: **02.01.2003**
(21) Application number: 98305095.6
(22) Date of filing: 29.06.1998
(51) Int. Cl.: A61L 15/18, A61L 15/24, A61P 17/02

(54) **Use of molecular sieves to promote chronic wound healing**
Verwendung von Molekularsieben zur Förderung der Heilung chronischer Wunden
Utilisation de tamis moléculaires pour favoriser la guérison des plaies chroniques

(30) Priority: 30.06.1997 GB 9713835
(43) Date of publication of application: 07.01.1999
(73) Proprietor: Johnson & Johnson Medical Ltd., Edinburgh EH2 4NH (GB)
(72) Inventor: Watt, Paul William, Steeton, West Yorkshire BD20 6UN (GB); Josephsen, Lotte, Riverside, Stirling FK8 1QS (GB); Grady, Michael William, Burley-in-Wharfedale LS29 7LP (GB); McGregor, James, Bishopbriggs, Glasgow G64 1AY (GB)
(74) Representative: Fisher, Adrian John

(56) References cited:
- EP-A- 0 103 214
- EP-A- 0 509 409
- WO-A-88/03397
- WO-A-97/49487
- GB-A- 2 265 314
- US-A- 4 822 349
- US-A- 4 826 497
- US-A- 5 120 693
- US-A- 5 474 545
- DATABASE WPI Section Ch, Week 8537 Derwent Publications Ltd., London, GB; Class A96, AN 85-223656 XP002083141 & DD 222 497 A (BEZIRKS DRESDEN-NEU) , 22 May 1985

## Description

The present invention relates to the use of molecular sieves for the preparation of a composition for application to a wound to promote wound healing of chronic wounds such as venous ulcers, diabetic ulcers and decubitis ulcers (pressure sores).

Currently preferred procedures for the treatment of chronic wounds, and in particular such common chronic wounds as venous ulcers, diabetic ulcers and pressure sores, include absorbent wound dressings and simple medicated wound dressings. None of these dressings has yet been optimised for chronic wound healing.

GB-A-2259858 describes the use of zeolites (a family of inorganic aluminosilicate molecular sieves) for the reduction of odor from malodorous wounds. The zeolite is sealed into a gas-permeable sachet, and the sachet containing the zeolite is placed over a wound dressing applied to a malodorous wound to absorb the malodorous substances emitted from the wound. There is no teaching of the application of the zeolite directly to the wound so that it contacts the wound fluid.

C. Christoforou *et al.* in Poster 148 presented at the 2nd Joint meeting of the Wound Healing Society and The European Tissue Repair Society, May 15th-19th 1996, in Boston, MA, USA, report that biodegradable, positively charged DEAE Sephadex ion exchange agarose beads have a positive effect on wound healing strength when applied to acute wounds *in vivo.*

The present invention provides the use of a molecular sieve material for the preparation of a composition for application to a wound to promote wound healing, wherein the molecular sieve material comprises microporous polymer beads, and the wound is a chronic wound selected from venous ulcers, diabetic ulcers and decubitis ulcers.

Without wishing to be bound by any theory, it is thought that the molecular sieve materials promote chronic wound healing by adsorption of matrix metalloproteinases (MMP's) from the wound fluid. The MMP's represent a family of structurally and functionally related enzymes responsible for proteolytic degradation of extracelluar matrix (ECM) components such as basement membrane. Dysregulation of MMP production and activation may cause altered ECM proteolysis, resulting in slow wound healing.

Preferably, the molecular sieve material used in the present invention is non-biodegradable. That is to say, the molecular sieve preferably does not break down *in vivo*. In contrast, certain biopolymers, such as collagens, that are known to bind MMP's, may exhibit reduced efficacy due to breakdown and release of bound MMP's *in vivo* when used as wound dressings.

Preferably, the molecular sieve material used in the present invention is non-swelling in water or serum. In contrast, certain biopolymer based ion exchange resins do hydrate to form gel-like matrices that inhibit the migration of small molecules, thereby inhibiting the efficiency with which MMP's are absorbed.

Preferably, the specific surface area of the molecular sieve material as determined by BET single point (dry sample) nitrogen adsorption methods is greater than 250 m²/g, preferably greater than 400 m²/g, more preferably greater than 500 m²/g, and most preferably greater than 750 m²/g.

Preferably, the molecular sieve consists essentially of polymer particles. Preferably, the polymer particles have a surface area of at least 750m²/g, more preferably at least 1000m²/g. In certain preferred embodiments, the molecular sieve is in the form of particles 10-150 µm in size.

Preferably, the polymer particles have a median pore diameter, as determined by mercury porosimetry, of less than 1.0µm, more preferably less than 0.25µm, and most preferably less than 0.1µm.

Preferably, the polymer particles comprise beads having diameters in the range 0.1-5.0mm, preferably 1.0-5.0mm.
Such beads may be commercially available for use as molecular sieves, or as the stationary phase for HPLC.

Preferably, the molecular sieve material comprises a non-biodegradable polymer that is non-swelling in water.

Preferably, the polymer particles comprise polystyrene, and more preferably the polymer particles consist essentially of cross-linked polystyrene. Suitable, highly porous cross-linked polystyrene particles are available under the Registered Trade Marks PUROSEP and MACRONETS from Purolite International Limited, Cowbridge Road, Pontyclun, Mid-Glamorgan, U.K. The Synthesis of these materials is described, for example, in SU-A-434757 and SU-A-804647. These materials have been used in the chemical engineering art as molecular sieves for the removal of impurities from liquids, but they have not hitherto been suggested for use in wound dressings.

The porous cross-linked polystyrene particles are formed by treatment of polystyrene, or its copolymers with 0.3-2% of divinylbenzene, with a bifunctional cross-linking agent in the presence of Friedel-Crafts catalysts in an organic solvent. The resulting cross-linked gel is washed and dried to provide the absorbent particles. Preferred bifunctional cross-linking agents include p-xylylene dichloride, monochlorodimethyl ether and dimethylformal. Preferred Friedel-Crafts catalysts include iron trichloride, aluminium trichloride, tin tetrachloride and zinc dichloride. Preferred organic solvents include dichloroethane, tetrachloroethane and nitrobenzene.

Preferably, the molecular sieve material is enclosed in a container formed wholly or in part from a woven, non-woven or knitted fabric. More preferably, the molecular sieve material is enclosed in an envelope, sleeve or sock formed entirely from the said fabric. Preferred fabrics are spun-bonded, non-woven, non-adherent nylon fabrics of the kind which are preferably held together by heat sealing. The resulting "tea-bag" containing the molecular sieve material is suitable for application directly to the surface of a wound, whereby the wound fluid is brought into contact with the molecular sieve material.

In other preferred embodiments the molecular sieve material is dispersed in a continuous solid phase of absorbent wound dressing material. Suitable materials include sponge materials, such as the cross-linked freeze-dried collagen sponge described in US-A-3157524 or WO 85/04413. In other preferred embodiments, the molecular sieve material is dispersed in a non-biodegradable absorbent matrix, such as the polyurethane sponge available from Johnson & Johnson Medical, Inc. under the Registered Trade Mark TIELLE.

Preferably, a layer comprising the molecular sieve material is combined with other layers in a multi-layer wound dressing. More preferably, the multi-layer wound dressing is sterile and packaged in a bacteria-impermeable container such as a plastics laminated foil sachet.

In accordance with the present invention, the molecular sieve material is applied to a chronic wound such as a venous ulcer, diabetic ulcer or decubitis ulcer, such that the wound fluid comes into contact with the molecular sieve material.

Specific embodiments of the present invention will now be described further, by way of example, with reference to the accompanying drawings, in which:
Figure 1 shows the amount of MMP activity detected in human acute wound fluid after incubation with and without (control) selected molecular sieve materials and ion exchange resins; and
Figure 2 shows the amount of MMP activity detected in SDS-sample buffer following protease extraction of MMP from the molecular sieves and ion exhange resins used in Figure 1.

### Procedure 1

A cross-linked polystyrene molecular sieve having very high internal surface area and uniform pore size is prepared as follows:

A solution of 2.6 g (0.01 mole) of SnCl₄ in 100 ml of tetrachloroethane is added to a solution of 104 g (1 mole) of polystyrene and 0.76 g (0.01 mole) of dimethylformal in 700 ml of the same solvent. The mixture is thoroughly stirred and held for 5 hours at room temperature. The resultant gel is broken up, washed with acetone, then with a mixture of acetone and 0.5N HCl, then with 0.5N HCl, then with water and the gel is then dried.

### Example 1

A wound dressing for use according to the present invention is prepared as follows.

Samples of PUROSEP (RTM) dark cross-linked microporous polystyrene beads obtained from the Purolite Company were sealed into nylon fabric bags by heat sealing. A number of such "tea bags" containing 150, 298 and 558 mg of the beads were prepared.

### Example 2

The extend of binding of pro MMP2 and pro MMP9 from human acute wound fluid to the wound dressings prepared in Example 1 was assessed as follows.

The bags from Example 1 were immersed in samples of 2.5 ml of a 1:10 dilution of mastectomy acute wound fluid. The samples were shaken in contact with the wound fluid for 3 hours at ambient temperature, followed by removal and rinsing of the tea-bag with SDS buffer.

The activity of the specified MMP proenzymes remaining in the wound fluid was then assessed by gelatin SDS polyacrylamide gel electrophoresis (zymography), as described by Heussen C. and Dowdle E.B.in Anal. Biochem. 102:196-202, (1980).

The area of the individual zones of clearance on the gels, which are due to proteinase activity, was measured by the OPTILAB (RTM) system. This was achieved by repeating each experiment (n=3) and analyzing the results statistically by the Students Test, where P is less than or equal to 0.05. The results were as follows:-

| Dressing | Pro9% of control | Pro2% of control |
|---|---|---|
| Nylon® | 100 | 100 |
| Nylon® + 158mg beads | 71 | 60 |
| Nylon® + 298mg beads | 57 | 34 |
| Nylon® + 558mg beads | 18.3 | 8.9 |

The strength of the MMP binding to the molecular sieves was assessed qualitatively as follows. The bags were removed and added to 5ml of MMP buffer and shaken for 1 hour to try to wash loosely bound proteins from the teabags. No protease activity was detected in the washes by SDS-PAGE gels.

The "tea-bags" were then treated with 2.5 ml of a Sample Buffer made up as follows: 6.3 ml of 0.5 m tris/HCl pH6.8 buffer, 2-5ml glycerol, 0.5g sodium dodecyl sulfonate (SDS), 16.2ml water. This strong detergent-based buffer also failed to strip any of the strongly-bound MMP from the molecular sieve.

### Example 3

A comparative study of MMP binding by a range of molecular sieve and ion exchange resin bead materials was performed as follows:

The following materials were selected for the study: Purposep (RTM) dark; Purosep (RTM) light, HISIV (RTM) 1000 zeolite, AG.50W zeolite; HISIV (RTM)3000 zeolite; gelatin agarose beads; QEA Sephadex (RTM) solid agarose beads supplied by Pharmacia Ltd; DEAE Sephadex (RTM) solid agarose beads supplied by Pharmacia Ltd; DEAE Agarose beads; and Dextran S04 beads.

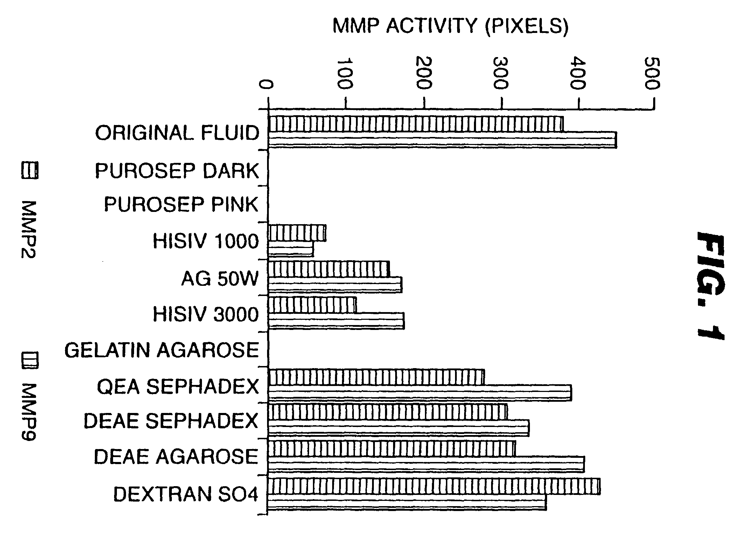

A sample of 100mgs of each material was placed in a microcentrifuge tube and lml of a 1:15 dilution of acute wound fluid added (Mastectomy fluid diluted in MMP buffer). The tubes were incubated for 3 hrs at room temperature, with shaking at speed 6 on an orbital mixer. After this time the tubes were spun at 1300rpm on the bench centrifuge and the supernatants collected and stored a -20°C. The pro MMP2 and pro MMP 9 activities remaining in 20µls aliquots of the supernatants were assessed by gelatin SDS polyacrylamide gel electrophoresis as described in Example 2, and the results are shown in Figure 1.

The results shown in Figure 1 demonstrate a clear reduction of MMP activity in the wound fluid samples that have been incubated with the molecular sieve materials. The reduction is most marked for the high surface area (greater than 800 m²/g) cross-linked polystyrene PUROSEP beads.

To each of the remaining residues in each tube was added 0.5ml of MMP buffer with brief mixing on the orbital mixer. Again these tubes were spun as before and the supernatant collected. This washing step should have liberated any weakly bound proteases from the various sample residues.

Finally, to each of the remaining residues in each tube was added 0.5ml of Sample Buffer (as defined above) with brief mixing on the orbital mixer.

These tubes were mixed gently overnight at 4°C on a shaker. The pro MMP2 and pro MMP9 activities in the supernatants (extracts) were then measured to assess the strength of the MMP binding to various materials.

20µls of the extracts were run on SDS-PAGE gelatin zymograms and the clearance caused by proteases was assessed by Image analysis system, NIH image by densitometry. Values are given as pixels of gelatin clearance on SDS-PAGE gels.

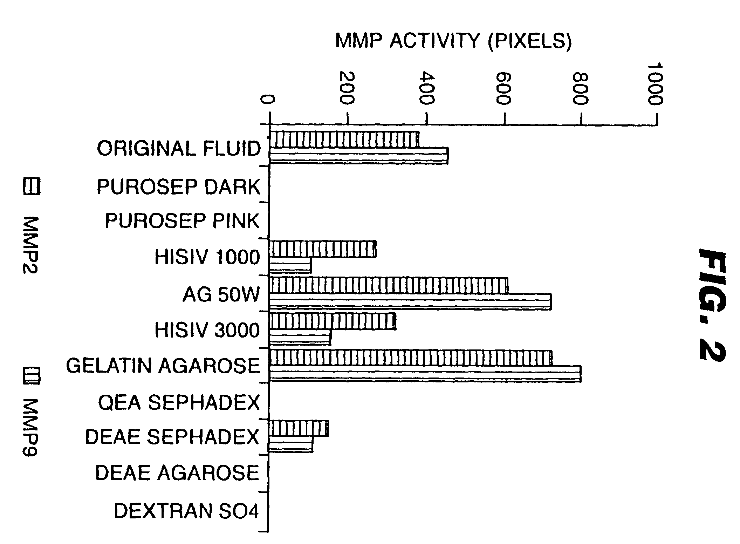

It can be seen from Figure 2 that some MMP can be extracted from the treated zeolite molecular sieves, but that the MMP is too strongly bound to the PUROSEP molecular sieves for extraction by this method.

Wound dressings containing the molecular sieves described above can be applied directly to the surface of chronic wounds to improve wound healing. Accordingly, in another aspect, the present invention provides a method of treating chronic wounds such as venous ulcers, pressure sores or diabetic ulcers, by applying thereto a wound dressing comprising a molecular sieve material.

## Claims

1. Use of a molecular sieve material for the preparation of a composition for application to a wound to promote wound healing, wherein the molecular sieve material comprises microporous polymer beads, and the wound is a chronic wound selected from venous ulcers, diabetic ulcers and decubitis ulcers.

2. Use according to claim 1, wherein the molecular sieve material comprises a non-biodegradable polymer that is non-swelling in water.

3. Use according to claim 2, wherein the molecular sieve material comprises cross-linked polystyrene beads.

4. Use according to any preceding claim, wherein the specific surface area of the molecular sieve material as determined by BET single point (dry sample) nitrogen adsorption measurements is greater than 250 m²/g.

5. Use according to claim 4, wherein the said specific surface area is greater than 400 m²/g.

6. Use according to any preceding claim, wherein the molecular sieve material is dispersed in an absorbent wound dressing material.

## Patentansprüche

1. Verwendung eines Molekularsiebmaterials zur Herstellung einer Zusammensetzung zur Aufbringung auf eine Wunde, um die Wundheilung zu fördern, wobei das Molekularsiebmaterial mikroporöse Polymerkügelchen umfaßt und die Wunde eine chronische Wunde ist, die unter Venengeschwüren, diabetischen Geschwüren und Wundliegegeschwüren ausgewählt ist.

2. Verwendung nach Anspruch 1, bei der das Molekularsiebmaterial ein nicht biologisch abbaubares Polymer ist, das in Wasser nicht quillt.

3. Verwendung nach Anspruch 2, bei der das Molekularsiebmaterial vernetzte Polystyrolkügelchen umfaßt.

4. Verwendung nach einem der vorangehenden Ansprüche, bei der das spezifische Oberflächengebiet des Molekularsiebmaterials, wie es durch Adsorptionsmessungen mit Stickstoff in einem Punkt (trockene Probe) gemäß BET bestimmt wird, größer als 250 m²/g ist.

5. Verwendung gemäß Anspruch 4, bei der das spezifische Oberflächengebiet größer als 400 m²/g ist.

6. Verwendung nach einem der vorangehenden Ansprüche, bei der das Molekularsiebmaterial in einem absorbierenden Wundverbandmaterial fein verteilt ist.

## Revendications

1. Utilisation d'une matière de type tamis moléculaire pour la préparation d'une composition pour application sur une plaie pour faciliter la cicatrisation, dans laquelle la matière de type tamis moléculaire comprend des billes de polymère microporeux et la plaie est une plaie chronique choisie parmi des ulcères veineux, des ulcères diabétiques et des ulcères de décubitus.

2. Utilisation selon la revendication 1, dans laquelle la matière de tamis moléculaire comprend un polymère non biodégradable qui ne gonfle pas dans l'eau.

3. Utilisation selon la revendication 2, dans laquelle la matière de tamis moléculaire comprend des billes de polystyrène réticulé.

4. Utilisation selon l'une quelconque des revendications précédentes, dans laquelle la superficie spécifique de la matière de type tamis moléculaire, déterminée par des mesures d'adsorption d'azote en un seul point BET (échantillon sec), est supérieure à 250 m²/g.

5. Utilisation selon la revendication 4, dans laquelle la superficie spécifique est supérieure à 400 m²/g.

6. Utilisation selon l'une quelconque des revendications précédentes, dans laquelle la matière de tamis moléculaire est dispersée dans une matière absorbante de pansement.
